# EUROPEAN PATENT APPLICATION

(11) **EP 4 393 383 A1**
(43) Date of publication of application: **03.07.2024**
(21) Application number: 22870081.1
(22) Date of filing: 11.05.2022
(51) Int. Cl.: A61B 5/021, A61B 5/00, A61H 31/00

(54) **APPARATUS FOR PROVIDING PREDICTED BLOOD PRESSURE DURING CARDIOPULMONARY RESUSCITATION, AND METHOD THEREFOR**

(30) Priority: 15.09.2021 KR 20210123000
(71) Applicant: University Industry Foundation, Yonsei University Wonju Campus, Gangwon-do 26493 (KR)
(72) Inventor: HWANG, Sung Oh, Wonju-si Gangwon-do 26386 (KR); HAN, Ji Ho, Wonju-si Gangwon-do 26493 (KR); YOON, Young Ro, Wonju-si Gangwon-do 26493 (KR); CHA, Kyoung Chul, Wonju-si Gangwon-do 26458 (KR)
(74) Representative: Vossius & Partner Patentanwälte Rechtsanwälte mbB
(86) International application number: PCT/KR2022/006756
(87) International publication number: WO 2023/042991

(57) **Abstract**

An apparatus for providing predicted blood pressure during cardiopulmonary resuscitation, and a method therefor are disclosed. An apparatus for providing predicted blood pressure during cardiopulmonary resuscitation, according to one embodiment of the present invention, comprises a blood pressure feedback device, which calculates index values from a chest compression waveform of a cardiopulmonary resuscitation feedback device and extracts predicted blood pressure on the basis of the calculated index values, so as to display or guide the predicted blood pressure, and thus an operator can be aware of a hemodynamic effect generated in a patent during cardiopulmonary resuscitation, so that the operator more effectively performs cardiopulmonary resuscitation.

## Description

### [Technical Field]

The present invention relates to a cardiopulmonary resuscitation feedback device, and more specifically, to an apparatus for providing predicted blood pressure during cardiopulmonary resuscitation and a method therefor which uses an index of a chest compression waveform to estimate and provide blood pressure generated due to chest compressions in a body of a cardiac arrest patient during cardiopulmonary resuscitation such that a hemodynamic effect (blood pressure) on the patient due to the chest compression can be known.

### [Background Art]

Cardiopulmonary resuscitation (CPR) feedback devices are devices that measure chest compressions and inform a rescuer of a chest compression state when the rescuer performs CPR on a cardiac arrest patient. The CPR feedback devices can primarily measure a rate and a depth of chest compressions by using a motion sensor or the like and can also secondarily calculate a period when chest compressions are interrupted and a compression fraction (a ratio of a chest compression time to a total time of CPR). The CPR feedback devices provide a rescuer with a visual or auditory feedback of a measured result. Commercialized feedback devices operate in a way of calculating an index related to a procedure of CPR and displaying the index on a monitor when a rescuer performs chest compressions with the devices placed between the sternum and a rescuer's hands or worn on the rescuer's wrist.

As described above, the CPR feedback devices inform a rescuer who performs CPR of a depth and a rate of chest compressions, thereby, fulfilling a function of guiding the rescuer to accurately perform chest compressions in accordance with the CPR guidelines. A reason for a rescuer to accurately perform chest compressions is to induce appropriate hemodynamic effects of keeping a patient alive with the rescuer's chest compressions.

Currently available CPR feedback devices inform characteristics (a compression depth, a compression rate, or the like) of chest compressions performed by a rescuer, but do not provide information on how high hemodynamic effects (systolic blood pressure, diastolic blood pressure, and mean blood pressure) of chest compressions a patient receives.

### [Citation List]

### [Patent Literature]

### [Patent Literature 1]

KR Patent Registration No. 101071432 (September 30, 2011)

### [Summary of Invention]

### [Technical Problem]

An object of the present invention to solve the problem described above is to provide an apparatus for providing predicted blood pressure during cardiopulmonary resuscitation (CPR) by predicting blood pressure based on a waveform generated due to chest compressions to provide a CPR provider with CPR feedback including hemodynamic effects such that the CPR provider can be aware of the hemodynamic effects (blood pressure) on a patient due to the chest compressions performed by the CPR provider.

### [Solution to Problem]

According to an embodiment of the present invention, an apparatus for providing predicted blood pressure during cardiopulmonary resuscitation can be realized by being configured to include a blood pressure feedback device that calculates index values from a chest compression waveform of a cardiopulmonary resuscitation feedback device and extracts predicted blood pressure on the basis of the calculated index values, so as to display or guide the predicted blood pressure.

The apparatus for providing predicted blood pressure during cardiopulmonary resuscitation may include: a pre-processing unit that analyzes the chest compression waveform of the cardiopulmonary resuscitation feedback device and calculates index values; a machine learning-based blood pressure prediction unit that calculates a systolic blood pressure value, a diastolic blood pressure value, and a mean predicted blood pressure value by using the index values calculated by the pre-processing unit; a neural network model-based point detection unit that predicts eight point values for generating a blood pressure waveform by using a neural network algorithm as an prediction model for multiple outputs using the index values calculated by the pre-processing unit; a blood pressure waveform generation unit that derives one segmented blood pressure waveform using spline interpolation from a total of eleven predicted values including the systolic blood pressure value, the diastolic blood pressure value, and the mean blood pressure value predicted by the machine learning-based blood pressure prediction unit and the eight point values detected by the neural network model-based point detection unit; and a control unit that displays, on a display, or provides voice guidance of one or more of the calculated predicted blood pressure values, the chest compression waveform, the blood pressure waveform, a chest compression depth, and a chest compression rate.

In addition, according to another embodiment of the present invention, an apparatus for providing predicted blood pressure during cardiopulmonary resuscitation that predicts, displays, and guides blood pressure by using a chest compression waveform of a cardiopulmonary resuscitation feedback device can be realized by being configured to include a blood pressure feedback device that calculates eleven point values from the chest compression waveform and generates a predicted blood pressure waveform from the calculated point values to display or guide the predicted blood pressure.

The blood pressure feedback device may be configured to include: a pre-processing unit that analyzes the chest compression waveform of the cardiopulmonary resuscitation feedback device and calculates index values; a machine learning-based blood pressure prediction unit that calculates three point values including a starting point, an ending point, and a peak point of a blood pressure segment by using the index values calculated by the pre-processing unit; a neural network model-based point detection unit that additionally derives eight point values by using the index values calculated by the pre-processing unit; a blood pressure waveform generation unit that calculates a predicted blood pressure waveform using spline interpolation between a total of the eleven points; and a control unit that displays, on a display, or provides voice guidance of one or more of the calculated predicted blood pressure waveform, predicted blood pressure, the chest compression waveform, a chest compression depth, and a chest compression rate.

According to still another embodiment of the present invention, a method for providing predicted blood pressure during cardiopulmonary resuscitation using a blood pressure feedback device that predicts, displays, and guides blood pressure by using a chest compression waveform of a cardiopulmonary resuscitation feedback device can be realized by being configured to include: a step of deriving a chest compression waveform from the cardiopulmonary resuscitation feedback device; a step of extracting signal pre-processing and input features to predict blood pressure through analysis of the derived chest compression waveform in the apparatus for providing predicted blood pressure during cardiopulmonary resuscitation; a step of predicting systolic blood pressure, diastolic blood pressure, and mean blood pressure as three main blood pressure values by using a GPR algorithm prediction model in the apparatus for providing predicted blood pressure using the input features extracted in the step; a step of predicting eight point values for generating a blood pressure waveform by using a neural network algorithm as a prediction model for multiple outputs in the apparatus for providing predicted blood pressure using the input features and the three blood pressure values extracted in the steps; a step of generating a blood pressure waveform by using the three predicted blood pressure values and the eight point values extracted in the steps in the apparatus for providing predicted blood pressure; and a step of controlling the chest compression waveform, the three predicted blood pressure values, and the blood pressure waveform obtained in the steps to be displayed.

### [Advantageous Effects of Invention]

Hence, according to the present invention, an apparatus for providing predicted blood pressure during cardiopulmonary resuscitation enables a cardiopulmonary resuscitation provider to be aware of a hemodynamic effect on a patient during cardiopulmonary resuscitation such that an effect of enabling the provider to more effectively perform cardiopulmonary resuscitation is achieved.

### [Brief Description of Drawings]

FIG. 1 is a view schematically illustrating an apparatus for predicting blood pressure during cardiopulmonary resuscitation according to the present invention.
FIG. 2 is a block diagram illustrating the apparatus for predicting blood pressure during cardiopulmonary resuscitation according to the present invention.
FIG. 3 is a flowchart for illustrating an operation of the apparatus for predicting blood pressure during cardiopulmonary resuscitation according to the present invention.
FIG. 4 is a graph illustrating a chest compression waveform and parameters.
FIG. 5 illustrates graphs for describing a method of generating a predicted blood pressure waveform.
FIG. 6 illustrates graphs for comparing a predicted blood pressure waveform and an actual blood pressure waveform.
FIG. 7 illustrates a concordance degree (left) of systolic blood pressure prediction using a blood pressure prediction indices and a Bland Altman plot (right) of prediction results.
FIG. 8 illustrates a concordance degree (left) of diastolic blood pressure prediction using a blood pressure prediction indices and a Bland Altman plot (right) of prediction results.
FIG. 9 illustrates a concordance degree (left) of mean arterial blood pressure prediction using a blood pressure prediction indices and a Bland Altman plot (right) of prediction results.

### [Description of Embodiments]

The terms or words used in the present specification and claims are not to be construed as being limited to usual or dictionary meanings and are to be construed as meanings and concepts consistent with the technical idea of the present invention based on a principle that the inventors can appropriately define concepts of the terms in order to explain his or her invention in the best way.

Throughout the specification, when a part is described to "comprise" a certain element, this means that the part may further include other elements rather than excluding other elements, unless specifically described to the contrary. In addition, terms such as "...unit", "... machine", "module", and "device" used in the specification refer to a unit that processes at least one function or operation, and this unit can be implemented by a combination of hardware and/or software.

Throughout the specification, the term "and/or" should be understood to include all possible combinations from one or more related items. For example, "a first item, a second item, and/or a third item" means not only the first, second, or third item but also combinations of all possible items that can be set from two or more items of the first, second, and third items.

Throughout the specification, identification codes (for example, a, b, c, ...) for steps are used for convenience of description and do not limit an order of the steps, and the steps may be performed in an order different from a specified order unless a specific order is clearly described in the context. That is, the steps may be performed in the same order as specified, may be performed substantially simultaneously, or may be performed in an opposite order.

Hereinafter, an embodiment of the present invention will be described with reference to the drawings.

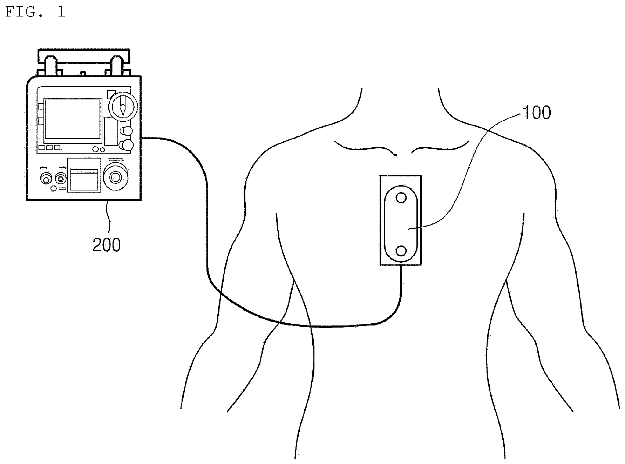

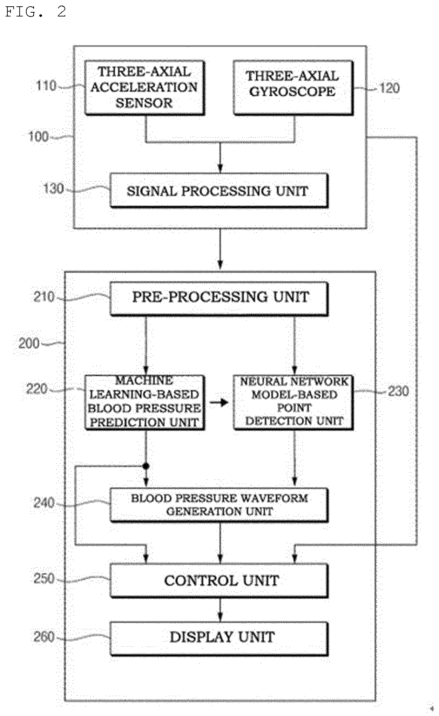

FIG. 1 is a view schematically illustrating an apparatus for predicting blood pressure during cardiopulmonary resuscitation according to the present invention, and FIG. 2 is a block diagram illustrating the apparatus for predicting blood pressure during cardiopulmonary resuscitation according to the present invention. As illustrated in the drawings, the apparatus for predicting blood pressure during cardiopulmonary resuscitation of the present invention includes an apparatus for predicting blood pressure during cardiopulmonary resuscitation 200 that predicts, displays, and guides blood pressure by using a chest compression waveform of a cardiopulmonary resuscitation feedback device 100.

The apparatus for predicting blood pressure during cardiopulmonary resuscitation 200 operates to calculate index values from a chest compression depth signal (hereinafter referred to as a chest compression waveform) fed back from the cardiopulmonary resuscitation feedback device 100 and extract predicted blood pressure by using the calculated index values to display or guide the predicted blood pressure.

In this respect, the cardiopulmonary resuscitation feedback device 100 can be defined as a device that is based on a three-axial acceleration sensor 110 and a three-axial gyroscope 120 and outputs a chest compression depth over time, and a common cardiopulmonary resuscitation feedback device can be used.

That is, various types of cardiopulmonary resuscitation feedback devices such as a type of device placed on a chest or worn on a wrist can be used, and a signal processing unit 130 receives a signal from the three-axial acceleration sensor 110 and the three-axial gyroscope 120 and obtains a chest compression waveform.

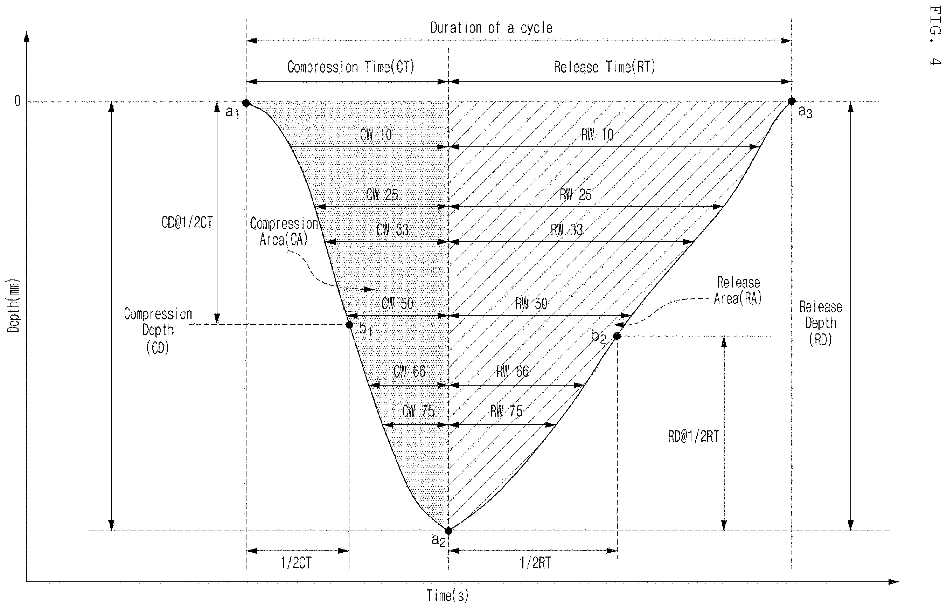

This chest compression waveform is illustrated in FIG. 4.

FIG. 4 is a graph illustrating the chest compression waveform (figure) and parameters generated by the cardiopulmonary resuscitation feedback device. The chest compression waveform includes index values such as a chest compression depth (CD), a chest compression time (CT), a mean compression velocity (Mean CV), a maximum compression velocity (Max CV), a release depth (RD), a release time (RT), a mean relaxation velocity (Mean RV), a maximum relaxation velocity (Max RV), a chest compression rate (CPR rate), or a compression/release time rate (Time rate) as index values for chest compression waveform analysis, and the index values are provided in a unit of a cycle.

Definitions of indices of the chest compression waveform in FIG. 4 are illustrated in Tables 1 and 2 below.

**[Table 1]**

| Index | Definition |
|---|---|
| Compression Depth (CD) | Chest compression depth |
| Compression Time (CT) | Chest compression time |
| 1/2CT Compression Distance (1/2CT CD) | Time until 1/2 of chest compression |
| Compression Width @ 10% of CD (CW10) | Width of chest compression waveform when chest compression depth reaches 10% |
| Compression Width @ 25% of CD (CW25) | Width of chest compression waveform when chest compression depth reaches 25% |
| Compression Width @ 33% of CD (CW33) | Width of chest compression waveform when chest compression depth reaches 33% |
| Compression Width @ 50% of CD (CW50) | Width of chest compression waveform when chest compression depth reaches 50% |
| Compression Width @ 66% of CD (CW66) | Width of chest compression waveform when chest compression depth reaches 66% |
| Compression Width @ 75% of CD (CW75) | Width of chest compression waveform when chest compression depth reaches 75% |
| Compression Area (CA) | Area under compression curve |
| Mean Compression Velocity (Mean CV) | Mean compression velocity |
| Max Compression Velocity (Max CV) | Maximum compression velocity |
| Compression Velocity 20 (CV20) | Compression velocity at 20% of compression time |
| Compression Velocity 40 (CV40) | Compression velocity at 40% of compression time |
| Compression Velocity 60 (CV60) | Compression velocity at 60% of compression time |
| Compression Velocity 80 (CV80) | Compression velocity at 80% of compression time |
| Release Depth (RD) | Release depth |
| Release Time (RT) | Release time |
| 1/2CT Relaxation Distance (1/2RT RD) | Time until 1/2 of chest relaxation |
| Relaxation Width @ 10% of RD (RW10) | Width of chest compression waveform when chest relaxation depth reaches 10% |
| Relaxation Width @ 25% of RD (RW25) | Width of chest compression waveform when chest relaxation depth reaches 25% |

**[Table 2]**

| | |
|---|---|
| Relaxation Width @ 33% of RD (RW33) | Width of chest compression waveform when chest relaxation depth reaches 33% |
| Relaxation Width @ 50% of RD (RW50) | Width of chest compression waveform when chest relaxation depth reaches 50% |
| Relaxation Width @ 66% of RD (RW66) | Width of chest compression waveform when chest relaxation depth reaches 66% |
| Relaxation Width @ 75% of RD (RW75) | Width of chest compression waveform when chest relaxation depth reaches 75% |
| Relaxation Area (RA) | Area under relaxation waveform |
| Mean Relaxation Velocity (Mean RV) | Mean relaxation velocity |
| Max Relaxation Velovity (Max RV) | Maximum relaxation velocity |
| Full Area (FA) | Full area under compression waveform |
| Relaxation Velocity 20 (RV20) | Compression velocity at 20% of relaxation time |
| Relaxation Velocity 40 (RV40) | Compression velocity at 40% of relaxation time |
| Relaxation Velocity 60 (RV60) | Compression velocity at 60% of relaxation time |
| Relaxation Velocity 80 (RV80) | Compression velocity at 80% of relaxation time |
| CPR rate | Chest compression rate |
| Time ratio | Ratio of compression time/relaxation time |
| Ex-Release Proportion | Incomplete relaxation proportion of previous compression waveform (indicating level of incomplete chest recoil after chest compression at previous chest compression waveform, when this value is 100, chest surface returns to original position of 0 after compression) |
| Release Proportion | Incomplete relaxation proportion of current compression waveform (indicating level of incomplete chest recoil after chest compression at current chest compression waveform, when this value is 100, chest surface returns to original position of 0 after compression) |
| Gender | Gender |
| Age | Age |

It is most desirable for the indices to include gender, age, and the like as in Table 2, but in the present invention, the indices for gender and age are to be reflected in the future.

In addition, the cardiopulmonary resuscitation feedback device 100 extracts chest compression elements including a chest compression depth, a chest compression rate, incomplete relaxation, a compression pause period, a compression fraction, or the like and transmits the chest compression elements to a control unit 250 of the apparatus for predicting blood pressure 200.

The apparatus for providing predicted blood pressure during cardiopulmonary resuscitation 200 is configured to predict blood pressure through signal analysis of the chest compression waveform received from the cardiopulmonary resuscitation feedback device 100, output analysis details of the predicted blood pressure, and visually display or provide voice guidance of the analysis details.

In addition, the apparatus for providing blood pressure 200 operates to calculate blood pressure values (predicted blood pressure: systolic blood pressure, diastolic blood pressure, and mean blood pressure) estimated by a machine learning method using the calculated index values.

It is needless to say that the apparatus for providing predicted blood pressure during cardiopulmonary resuscitation 200 can be configured as a module-type electronic device that can be used independently or in combination with a common cardiopulmonary resuscitation feedback device which outputs a chest compression depth. An electronic system for performing a prediction algorithm can be configured internally.

In addition, the apparatus for providing predicted blood pressure during cardiopulmonary resuscitation 200 of the present invention can provide not only a blood pressure prediction result in real time but also an analysis (cardiopulmonary resuscitation result for each time and each operator) result for debriefing a cardiopulmonary resuscitation result after cardiopulmonary resuscitation is ended.

In this respect, the apparatus for providing predicted blood pressure during cardiopulmonary resuscitation 200 includes a pre-processing unit 210 that pre-processes the chest compression waveform received from the cardiopulmonary resuscitation feedback device 100 by receiving, as digital signals, changes in the chest compression depth in real time over time.

The pre-processing unit 210 has functions of removing noise or redundant signals from the signals and segmenting the signals to input the segmented signals into a prediction model and extracts indices from the segmented chest compression signal. The extracted indices are used as input values to the prediction model.

Feature values extracted from the pre-processing unit 210 are input into two prediction models, respectively.

That is, a machine learning-based blood pressure prediction unit 220 operates to predict systolic blood pressure, diastolic blood pressure, and mean blood pressure as three main blood pressure values by using a GPR algorithm prediction model.

Since the systolic and diastolic blood pressure values are important values, a highly accurate GPR algorithm is used.

A regression model trained with a machine learning technology with an algorithm for predicting the blood pressure is based on the GPR algorithm, and a task of transplanting the regression model to a platform suitable for use in real life is carried out in a smooth laboratory environment. In addition, new data is acquired and is input into a previously trained model to be trained again, and thereby prediction ability can be improved.

In addition, the machine learning-based blood pressure prediction unit 220 is trained to extract a correlation between predicted blood pressure and the chest compression depth and rate and operates to predict the depth and rate according to the predicted blood pressure.

Moreover, a neural network model-based point detection unit 230 predicts eight point values for generating a blood pressure waveform by using a neural network algorithm as a prediction model for multiple outputs.

Each prediction model is a model which is trained in advance based on a large amount of CPR data and is designed to have high prediction accuracy. Hence, when new data is input, the prediction model operates to gradually increase prediction accuracy through training.

In an actual medical situation, systolic and diastolic blood pressure values are important monitoring elements of CPR and are elements which have to be displayed.

A blood pressure waveform generation unit 240 operates to receive systolic blood pressure, diastolic blood pressure, and mean blood pressure as inputs from the machine learning-based blood pressure prediction unit 220 and receive, as an input, the eight point values for generating the predicted blood pressure waveform predicted from the neural network model-based point detection unit 230 to predict a blood pressure waveform.

That is, the blood pressure waveform generation unit 240 derives one segmented blood pressure waveform using spline interpolation from a total of eleven predicted values and transmits the segmented blood pressure waveform to the control unit 250.

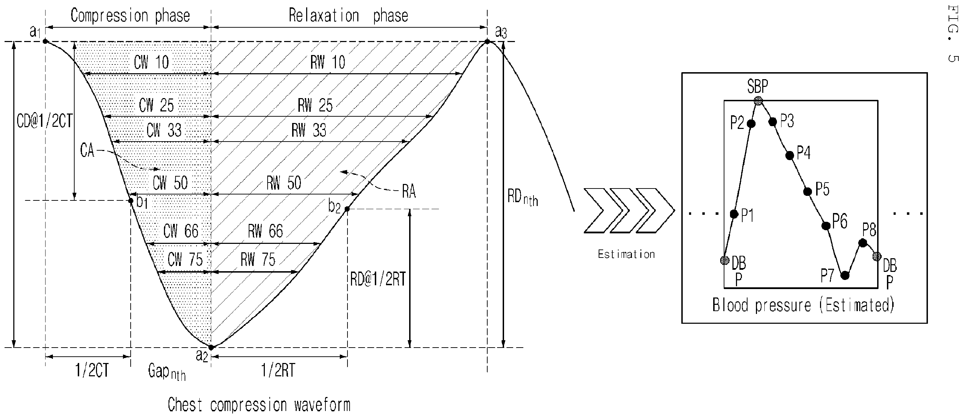

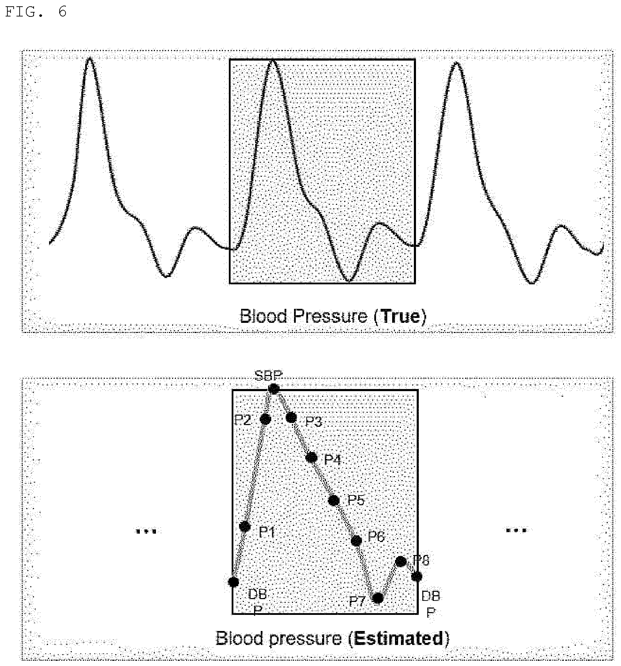

With reference to a reference drawing for describing a method of generating the predicted blood pressure waveform in FIG. 5 and graphs for comparing a predicted blood pressure waveform and an actual blood pressure waveform in FIG. 6, the systolic blood pressure and diastolic blood pressure enable a starting point, an ending point, and a peak point of a blood pressure segment to be illustrated.

In the drawing, DBP (diastolic blood pressure) and SBP (systolic blood pressure) are represented by blue dots.

The remaining eight points are defined as values at the time when a width (time) of the blood pressure segment is divided into eight equal parts, and the eight values are input into the trained neural network algorithm for multiple outputs to derive values. In the drawing, the values are represented by black dots as P1 to P8.

Consequently, a total of eleven points are generated, and a space between these points can be filled with data using spline interpolation.

With reference to FIG. 6, a lower figure corresponds to the blood pressure waveform generated by the apparatus for providing predicted blood pressure of the present invention, and the upper figure corresponds to an actual blood pressure waveform.

Hence, the apparatus for providing predicted blood pressure of the present invention can be versatilely combined with the cardiopulmonary resuscitation feedback device to predict continuous blood pressure values in a non-invasive manner.

In addition, the apparatus for providing predicted blood pressure of the present invention can provide hemodynamic feedback (blood pressure and blood pressure waveform) in addition to values (the compression depth and rate, incomplete relaxation, the chest compression pause period, and the chest compression fraction) calculated from existing chest compression waveforms.

In addition, the blood pressure waveform generated by the blood pressure waveform generation unit 240 and the systolic blood pressure, the diastolic blood pressure, and the mean blood pressure predicted by the machine learning-based blood pressure prediction unit 220 are displayed on a final display unit 260 such that the chest compression waveform generated by the cardiopulmonary resuscitation feedback device 100 can be visually displayed.

The control unit 250 controls each component of the apparatus for providing predicted blood pressure 200 and controls, in particular, the display unit 260 to receive the blood pressure waveform generated by the blood pressure waveform generation unit 240 and the correlation between the systolic blood pressure, the diastolic blood pressure, and the mean blood pressure and the chest compression depth and rate predicted by the machine learning-based blood pressure prediction unit 220 and display or provide voice guidance of chest compression elements including the chest compression depth and rate, the incomplete relaxation, the compression pause period, the compression fraction, or the like transmitted from the cardiopulmonary resuscitation feedback device 100.

Consequently, the display unit 260 displays information on the chest compression waveform, the predicted blood pressure value according to the chest compression waveform, and the chest compression depth and rate according to the predicted blood pressure value.

Hence, by using this information, a CPR provider can be aware of visually or by voice the hemodynamic effects on a patient during CPR, and thereby the CPR provider can perform CPR more effectively.

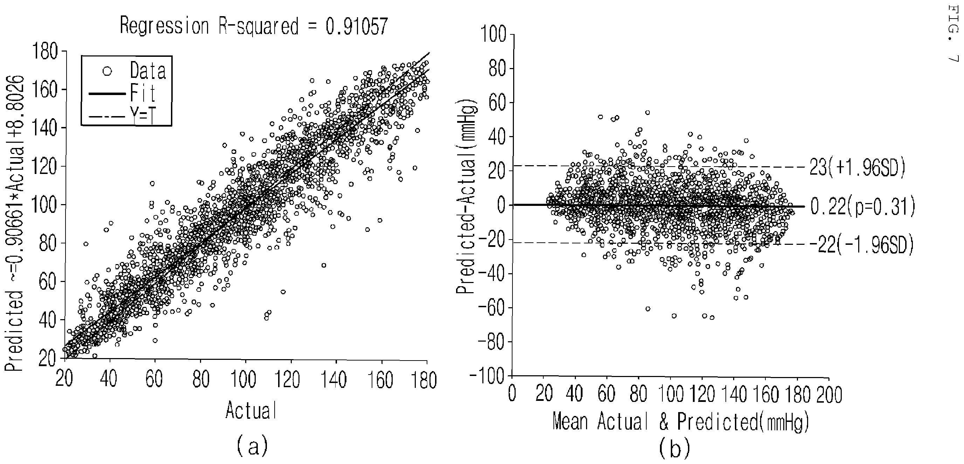

Table 3 and FIGS. 7 to 9 illustrate the results of prediction concordance degree analysis using blood pressure prediction indices, and Table 3 illustrates performance of regression models trained through machine learning for each feature selection algorithm.

**[Table 3]**

| Feature selection algorithm | Result determination index | GPR | | |
|---|---|---|---|---|
| | | Systolic | Diastolic | Mean arterial blood pressure |
| All Features | MAE | 9.43 | 3.10 | 2.91 |
| | MSE | 167.51 | 20.0 | 18.70 |
| | RMSE | 12.94 | 4.47 | 4.32 |
| | R-Square | 0.89 | 0.84 | 0.9 |
| ReliefF | MAE | 8.914 | 3.07 | 2.91 |
| | MSE | 152.98 | 19.7 | 18.92 |
| | RMSE | 12.36 | 4.44 | 0.9 |
| | R-Square | 0.9 | 0.84 | 0.9 |
| MRMR | MAE | 9.45 | 3.15 | 2.96 |
| | MSE | 171.01 | 21.01 | 19.05 |
| | RMSE | 13.07 | 4.58 | 4.36 |
| | R-Square | 0.88 | 0.83 | 0.9 |
| NCA | MAE | 8.68 | 3.08 | 2.77 |
| | MSE | 148.24 | 19.67 | 17.18 |
| | RMSE | 12.18 | 4.43 | 4.14 |
| | R-Square | 0.9 | 0.84 | 0.91 |

Table 3 shows that the most accurate prediction ability is achieved by selecting features using an NCA feature selection algorithm and a combination of GPR regression models.

Hence, the machine learning-based blood pressure prediction unit 220 of the present invention is exemplified to extract the three main blood pressure values using the GPR algorithm prediction model.

FIGS. 7 to 9 illustrate results of concordance degree analysis of arterial blood pressure prediction using blood pressure prediction indices. FIGS. 7 to 9 illustrate the prediction performance as graphs by inputting, into a regression model, data which is put aside without being used for learning. (a) of FIG. 7 illustrates a concordance degree of systolic blood pressure prediction using the blood pressure prediction indices, and (b) of FIG. 7 illustrates a Bland Altman plot of the prediction results.

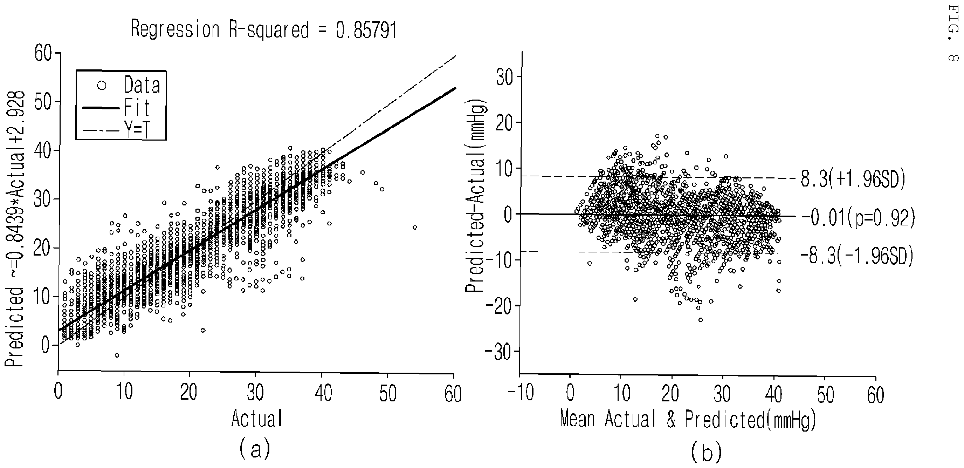

In addition, (a) of FIG. 8 illustrates a concordance degree of diastolic blood pressure prediction using the blood pressure prediction indices, and (b) of FIG. 8 illustrates a Bland Altman plot of the prediction results.

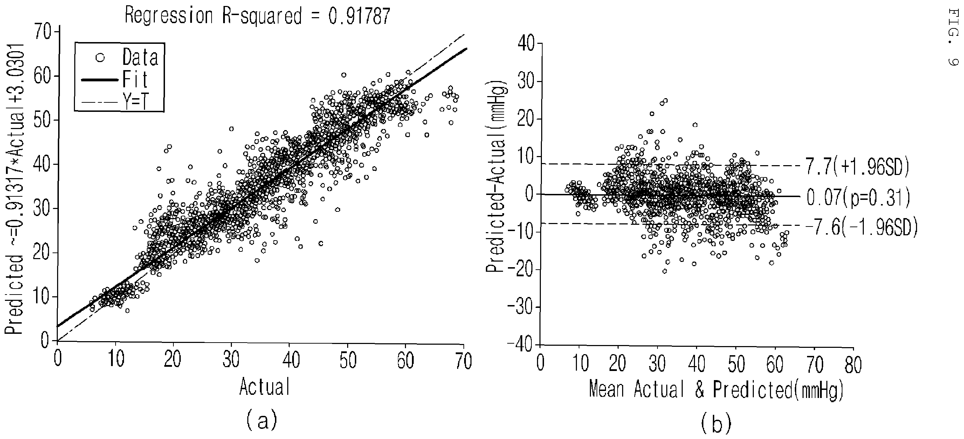

In addition, (a) of FIG. 9 illustrates a concordance degree of mean arterial blood pressure prediction using the blood pressure prediction indices, and (b) of FIG. 9 illustrates a Bland Altman plot of the prediction results.

Hereinafter, with reference to the drawings, an operation of the device that has the above-described configuration and predicts blood pressure during cardiopulmonary resuscitation through chest compression waveform analysis will be described.

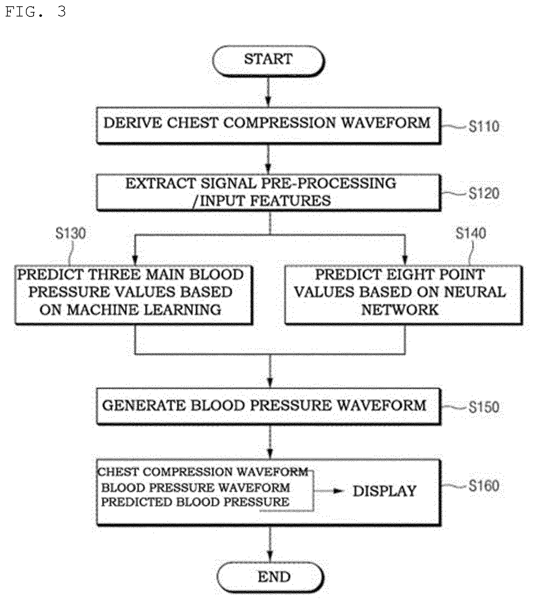

FIG. 3 is a flowchart for illustrating the operation of the apparatus for predicting blood pressure during cardiopulmonary resuscitation according to the present invention. As illustrated in the drawing, the apparatus for predicting blood pressure during cardiopulmonary resuscitation of the present invention operates to receive, as the input, the chest compression waveform from the cardiopulmonary resuscitation feedback device 100 and causes the apparatus for providing predicted blood pressure 200 to predict, display, and guide blood pressure.

Specifically, the chest compression waveform is derived from the cardiopulmonary resuscitation feedback device 100 (S110).

The chest compression waveform derived in step S110 includes index values such as a chest compression depth (CD), a chest compression time (CT), a mean compression velocity (Mean CV), a maximum compression velocity (Max CV), a release depth (RD), a release time (RT), a mean relaxation velocity (Mean RV), a maximum relaxation velocity (Max RV), a chest compression rate (CPR rate), and a compression/release time rate (Time rate) as index values for chest compression waveform analysis.

In addition, the cardiopulmonary resuscitation feedback device 100 extracts the chest compression elements including a chest compression depth, a chest compression rate, incomplete relaxation, a compression pause period, a compression fraction, or the like and transmits the chest compression elements to the apparatus for providing predicted blood pressure 200.

The apparatus for providing predicted blood pressure during cardiopulmonary resuscitation 200 extracts the signal pre-processing and input features to predict blood pressure through analysis of the chest compression waveform derived in step S110 (S120).

Specifically, the pre-processing unit 210 receives, as digital signals, changes in the chest compression depth in real time over time and pre-processes the changes.

In addition, the pre-processing unit 210 has functions of removing noise or redundant signals from the signals and segmenting the signals to input the segmented signals into a prediction model and extracts indices from the segmented chest compression signal. Regarding extracted features, input features are extracted to be used as input values in the prediction model.

The feature values extracted in step S120 are respectively input to two prediction models (S130, 5140).

That is, in step S130, the machine learning-based blood pressure prediction unit 220 predicts the systolic blood pressure, the diastolic blood pressure, and the mean blood pressure as three main blood pressure values by using the GPR algorithm prediction model.

In addition, the machine learning-based blood pressure prediction unit 220 is trained to extract a correlation between predicted blood pressure and the chest compression depth and rate and operates to predict the depth and rate according to the predicted blood pressure.

In step S140, the neural network model-based point detection unit 230 predicts the eight point values for generating the blood pressure waveform by using a neural network algorithm as a prediction model for multiple outputs.

The blood pressure waveform generation unit 240 generates the blood pressure waveform by using the three predicted blood pressure values and the eight point values in steps S130 and S140 (S150).

That is, the blood pressure waveform generation unit 240 derives one segmented blood pressure waveform using spline interpolation from a total of eleven predicted values and transmits the segmented blood pressure waveform to the control unit 250.

The control unit 250 controls the display unit 260 to display or provide voice guidance of the blood pressure waveform generated by the blood pressure waveform generation unit 240, the systolic blood pressure, the diastolic blood pressure, and the mean blood pressure predicted by the machine learning-based blood pressure prediction unit 220, and the chest compression elements including the chest compression depth and rate, the incomplete relaxation, the compression pause period, the compression fraction, or the like transmitted from the cardiopulmonary resuscitation feedback device 100 (S160).

By modularizing the above-described device that predicts blood pressure during CPR through the chest compression waveform analysis, the device can be commercialized as an element of a defibrillator/monitor using a cardiopulmonary resuscitation feedback device.

Although the present invention has been described in detail with respect to the embodiments described above, it is obvious to those skilled in the art that various changes and modifications are possible within the technical scope of the present invention, and it is natural that the changes and modifications fall within the scope of the accompanying claims.

### [Industrial Applicability]

The present invention is implemented as a device that predicts and provides, by using an index of a chest compression waveform, blood pressure generated due to chest compressions in a body of a cardiac arrest patient during CPR to provide predicted blood pressure during CPR so as to a CPR provider to perform CPR more effectively, but the present invention can be applied to various industrial fields within a range in which the same configuration as the present invention is employed.

## Claims

1. An apparatus for providing predicted blood pressure during cardiopulmonary resuscitation that predicts, displays, and guides blood pressure by using a chest compression waveform of a cardiopulmonary resuscitation feedback device, comprising:
a blood pressure feedback device that calculates index values from the chest compression waveform, extracts predicted blood pressure with the calculated index values, and displays or guides the predicted blood pressure.

2. The apparatus for providing predicted blood pressure during cardiopulmonary resuscitation claim 1,
wherein the cardiopulmonary resuscitation feedback device extracts a depth and a rate of chest compressions and transmits the depth and the rate to the blood pressure feedback device, and
the blood pressure feedback device displays or guides the chest compression waveform, the predicted blood pressure, and the depth and the rate of chest compressions depending on the predicted blood pressure.

3. The apparatus for providing predicted blood pressure during cardiopulmonary resuscitation according to claim 2,
wherein the blood pressure feedback device includes:
a pre-processing unit that analyzes the chest compression waveform of the cardiopulmonary resuscitation feedback device and calculates index values;
a machine learning-based blood pressure prediction unit that calculates a systolic blood pressure value, a diastolic blood pressure value, and a mean predicted blood pressure value by using the index values calculated by the pre-processing unit;
a neural network model-based point detection unit that predicts eight point values for generating a blood pressure waveform by using a neural network algorithm as a prediction model for multiple outputs using the index values calculated by the pre-processing unit;
a blood pressure waveform generation unit that derives one segmented blood pressure waveform using spline interpolation from a total of eleven predicted values including the systolic blood pressure value, the diastolic blood pressure value, and the mean blood pressure value predicted by the machine learning-based blood pressure prediction unit and the eight point values detected by the neural network model-based point detection unit; and
a control unit that displays, on a display, or provides voice guidance of one or more of the calculated predicted blood pressure values, the chest compression waveform, the blood pressure waveform, a chest compression depth, and a chest compression rate.

4. The apparatus for providing predicted blood pressure during cardiopulmonary resuscitation according to claim 3,
wherein the pre-processing unit calculates two or more values of a chest compression depth (CD), a chest compression time (CT), a mean compression velocity (Mean CV), a maximum compression velocity (Max CV), a release depth (RD), a release time (RT), a mean relaxation velocity (Mean RV), a maximum relaxation velocity (Max RV), a chest compression rate (CPR rate), and a compression/release time rate (Time rate) as index values for chest compression waveform analysis from the chest compression waveform of the CPR feedback device.

5. The apparatus for providing predicted blood pressure during cardiopulmonary resuscitation according to claim 3,
wherein the machine learning-based blood pressure prediction unit calculates index values from the chest compression waveform and trains and stores predicted blood pressure for the calculated index values.

6. An apparatus for providing predicted blood pressure during cardiopulmonary resuscitation that predicts, displays, and guides blood pressure by using a chest compression waveform of a cardiopulmonary resuscitation feedback device, comprising:
a blood pressure feedback device that calculates eleven point values from the chest compression waveform and generates a predicted blood pressure waveform from the calculated point values to display or guide the predicted blood pressure.

7. The apparatus for providing predicted blood pressure during cardiopulmonary resuscitation according to claim 6,
wherein the blood pressure feedback device includes:
a pre-processing unit that analyzes the chest compression waveform of the cardiopulmonary resuscitation feedback device and calculates index values;
a machine learning-based blood pressure prediction unit that calculates three point values including a starting point, an ending point, and a peak point of a blood pressure segment by using the index values calculated by the pre-processing unit;
a neural network model-based point detection unit that additionally derives eight point values by using the index values calculated by the pre-processing unit;
a blood pressure waveform generation unit that calculates a predicted blood pressure waveform by using spline interpolation between a total of the eleven points; and
a control unit that displays, on a display, or provides voice guidance of one or more of the calculated predicted blood pressure waveform, predicted blood pressure, the chest compression waveform, a chest compression depth, and a chest compression rate.

8. The apparatus for providing predicted blood pressure during cardiopulmonary resuscitation according to claim 7,
wherein the pre-processing unit calculates two or more values of a chest compression depth (CD), a chest compression time (CT), a mean compression velocity (Mean CV), a maximum compression velocity (Max CV), a release depth (RD), a release time (RT), a mean relaxation velocity (Mean RV), a maximum relaxation velocity (Max RV), a chest compression rate (CPR rate), and a compression/release time rate (Time rate) as index values for chest compression waveform analysis from the chest compression waveform of the CPR feedback device.

9. The apparatus for providing predicted blood pressure during cardiopulmonary resuscitation according to claim 7,
wherein the neural network model-based point detection unit calculates three point values of a starting point, an ending point, and a peak point from the compression waveform analysis, displays the point values on the predicted blood pressure waveform and derives additional eight point values by horizontally dividing the starting point and the ending point into eight parts.

10. A method for providing predicted blood pressure during cardiopulmonary resuscitation using a blood pressure feedback device that predicts, displays, and guides blood pressure by using a chest compression waveform of a cardiopulmonary resuscitation feedback device, comprising:
(a) deriving a chest compression waveform from the cardiopulmonary resuscitation feedback device;
(b) extracting signal pre-processing and input features to predict blood pressure through analysis of the chest compression waveform derived from the apparatus for providing predicted blood pressure during cardiopulmonary resuscitation;
(c) predicting systolic blood pressure, diastolic blood pressure, and mean blood pressure as three main blood pressure values by using a GPR algorithm prediction model in the apparatus for providing predicted blood pressure using the input features extracted in (b);
(d) predicting eight point values for generating a blood pressure waveform by using a neural network algorithm as a prediction model for multiple outputs in the apparatus for providing predicted blood pressure using the input features extracted in (b) and the three blood pressure values extracted in (c);
(e) generating a blood pressure waveform by using the three predicted blood pressure values extracted in (c) and the eight point values extracted in (d) in the apparatus for providing predicted blood pressure; and
(f) controlling the chest compression waveform derived in (a), the three predicted blood pressure values obtained in (c), and the blood pressure waveform generated in (e) to be displayed on the apparatus for providing predicted blood pressure.

11. The method for providing predicted blood pressure during cardiopulmonary resuscitation according to claim 10,
wherein (a) further includes extracting a chest compression element including a chest compression depth, a chest compression rate, incomplete relaxation, a compression pause period, and a compression fraction from the chest compression waveform and transmitting the chest compression element to the apparatus for providing predicted blood pressure during cardiopulmonary resuscitation.

12. The method for providing predicted blood pressure during cardiopulmonary resuscitation according to claim 10,
wherein, in (a), the chest compression waveform includes two or more index values of a chest compression depth (CD), a chest compression time (CT), a mean compression velocity (Mean CV), a maximum compression velocity (Max CV), a release depth (RD), a release time (RT), a mean relaxation velocity (Mean RV), a maximum relaxation velocity (Max RV), a chest compression rate (CPR rate), and a compression/release time rate (Time rate) as index values for chest compression waveform analysis.

13. The method for providing predicted blood pressure during cardiopulmonary resuscitation according to claim 10,
wherein (c) further includes performing training to extract a correlation between predicted blood pressure and chest compression depth and rate and predicting a depth and a rate depending on predicted blood pressure.

14. The method for providing predicted blood pressure during cardiopulmonary resuscitation according to claim 10,
wherein (e) further includes deriving one segmented blood pressure waveform from the three predicted blood pressure values and the eight point values by using spline interpolation.

15. The method for providing predicted blood pressure during cardiopulmonary resuscitation according to claim 11,
wherein (f) includes displaying the chest compression depth, the chest compression rate, the incomplete relaxation, the compression pause period, and the compression fraction transmitted in (a) to display predicted blood pressure, a chest compression depth, and a chest compression rate.

## Amended claims

### Amended claims under Art. 19.1 PCT

1. An apparatus for providing predicted blood pressure during cardiopulmonary resuscitation that predicts, displays, and guides blood pressure by using a chest compression waveform of a cardiopulmonary resuscitation feedback device, comprising:
a blood pressure feedback device that calculates index values from the chest compression waveform, extracts predicted blood pressure with the calculated index values, and displays or guides the predicted blood pressure.

2. The apparatus for providing predicted blood pressure during cardiopulmonary resuscitation claim 1,
wherein the cardiopulmonary resuscitation feedback device extracts a depth and a rate of chest compressions and transmits the depth and the rate to the blood pressure feedback device, and
the blood pressure feedback device displays or guides the chest compression waveform, the predicted blood pressure, and the depth and the rate of chest compressions depending on the predicted blood pressure.

3. The apparatus for providing predicted blood pressure during cardiopulmonary resuscitation according to claim 2,
wherein the blood pressure feedback device includes:
a pre-processing unit that analyzes the chest compression waveform of the cardiopulmonary resuscitation feedback device and calculates index values;
a machine learning-based blood pressure prediction unit that calculates a systolic blood pressure value, a diastolic blood pressure value, and a mean predicted blood pressure value by using the index values calculated by the pre-processing unit;
a neural network model-based point detection unit that predicts eight point values for generating a blood pressure waveform by using a neural network algorithm as a prediction model for multiple outputs using the index values calculated by the pre-processing unit;
a blood pressure waveform generation unit that derives one segmented blood pressure waveform using spline interpolation from a total of eleven predicted values including the systolic blood pressure value, the diastolic blood pressure value, and the mean blood pressure value predicted by the machine learning-based blood pressure prediction unit and the eight point values detected by the neural network model-based point detection unit; and
a control unit that displays, on a display, or provides voice guidance of one or more of the calculated predicted blood pressure values, the chest compression waveform, the blood pressure waveform, a chest compression depth, and a chest compression rate.

4. The apparatus for providing predicted blood pressure during cardiopulmonary resuscitation according to claim 3,
wherein the pre-processing unit calculates two or more values of a chest compression depth (CD), a chest compression time (CT), a mean compression velocity (Mean CV), a maximum compression velocity (Max CV), a release depth (RD), a release time (RT), a mean relaxation velocity (Mean RV), a maximum relaxation velocity (Max RV), a chest compression rate (CPR rate), and a compression/release time rate (Time rate) as index values for chest compression waveform analysis from the chest compression waveform of the CPR device.

5. The apparatus for providing predicted blood pressure during cardiopulmonary resuscitation according to claim 3,
wherein the machine learning-based blood pressure prediction unit calculates index values from the chest compression waveform and trains and stores predicted blood pressure for the calculated index values.

6. An apparatus for providing predicted blood pressure during cardiopulmonary resuscitation that predicts, displays, and guides blood pressure by using a chest compression waveform of a cardiopulmonary resuscitation feedback device, comprising:
a blood pressure feedback device that calculates eleven point values from the chest compression waveform and generates a predicted blood pressure waveform from the calculated point values to display or guide the predicted blood pressure.

7. The apparatus for providing predicted blood pressure during cardiopulmonary resuscitation according to claim 6,
wherein the blood pressure feedback device includes:
a pre-processing unit that analyzes the chest compression waveform of the cardiopulmonary resuscitation feedback device and calculates index values;
a machine learning-based blood pressure prediction unit that calculates three point values including a starting point, an ending point, and a peak point of a blood pressure segment by using the index values calculated by the pre-processing unit;
a neural network model-based point detection unit that additionally derives eight point values by using the index values calculated by the pre-processing unit;
a blood pressure waveform generation unit that calculates a predicted blood pressure waveform by using spline interpolation between a total of the eleven points; and
a control unit that displays, on a display, or provides voice guidance of one or more of the calculated predicted blood pressure waveform, predicted blood pressure, the chest compression waveform, a chest compression depth, and a chest compression rate.

8. The apparatus for providing predicted blood pressure during cardiopulmonary resuscitation according to claim 7,
wherein the pre-processing unit calculates two or more values of a chest compression depth (CD), a chest compression time (CT), a mean compression velocity (Mean CV), a maximum compression velocity (Max CV), a release depth (RD), a release time (RT), a mean relaxation velocity (Mean RV), a maximum relaxation velocity (Max RV), a chest compression rate (CPR rate), and a compression/release time rate (Time rate) as index values for chest compression waveform analysis from the chest compression waveform of the CPR device.

9. The apparatus for providing predicted blood pressure during cardiopulmonary resuscitation according to claim 7,
wherein the neural network model-based point detection unit calculates three point values of a starting point, an ending point, and a peak point from the compression waveform analysis, displays the point values on the predicted blood pressure waveform and derives additional eight point values by horizontally dividing the starting point and the ending point into eight parts.

10. A method for providing predicted blood pressure during cardiopulmonary resuscitation using a blood pressure feedback device that predicts, displays, and guides blood pressure by using a chest compression waveform of a cardiopulmonary resuscitation feedback device, comprising:
(a) deriving a chest compression waveform from the cardiopulmonary resuscitation feedback device;
(b) extracting signal pre-processing and input features to predict blood pressure through analysis of the chest compression waveform derived from the apparatus for providing predicted blood pressure during cardiopulmonary resuscitation;
(c) predicting systolic blood pressure, diastolic blood pressure, and mean blood pressure as three main blood pressure values by using a GPR algorithm prediction model in the apparatus for providing predicted blood pressure using the input features extracted in (b);
(d) predicting eight point values for generating a blood pressure waveform by using a neural network algorithm as a prediction model for multiple outputs in the apparatus for providing predicted blood pressure using the input features extracted in (b) and the three blood pressure values extracted in (c);
(e) generating a blood pressure waveform by using the three predicted blood pressure values extracted in (c) and the eight point values extracted in (d) in the apparatus for providing predicted blood pressure; and
(f) controlling the chest compression waveform derived in (a), the three predicted blood pressure values obtained in (c), and the blood pressure waveform generated in (e) to be displayed on the apparatus for providing predicted blood pressure.

11. The method for providing predicted blood pressure during cardiopulmonary resuscitation according to claim 10,
wherein (a) further includes extracting a chest compression element including a chest compression depth, a chest compression rate, incomplete relaxation, a compression pause period, and a compression fraction from the chest compression waveform and transmitting the chest compression element to the apparatus for providing predicted blood pressure during cardiopulmonary resuscitation.

12. The method for providing predicted blood pressure during cardiopulmonary resuscitation according to claim 10,
wherein, in (a), the chest compression waveform includes two or more index values of a chest compression depth (CD), a chest compression time (CT), a mean compression velocity (Mean CV), a maximum compression velocity (Max CV), a release depth (RD), a release time (RT), a mean relaxation velocity (Mean RV), a maximum relaxation velocity (Max RV), a chest compression rate (CPR rate), and a compression/release time rate (Time rate) as index values for chest compression waveform analysis.

13. The method for providing predicted blood pressure during cardiopulmonary resuscitation according to claim 10,
wherein (c) further includes performing training to extract a correlation between predicted blood pressure and chest compression depth and rate and predicting a depth and a rate depending on predicted blood pressure.

14. The method for providing predicted blood pressure during cardiopulmonary resuscitation according to claim 10,
wherein (e) further includes deriving one segmented blood pressure waveform from the three predicted blood pressure values and the eight point values by using spline interpolation.

15. The method for providing predicted blood pressure during cardiopulmonary resuscitation according to claim 11,
wherein (f) includes displaying the chest compression depth, the chest compression rate, the incomplete relaxation, the compression pause period, and the compression fraction transmitted in (a) to display predicted blood pressure, a chest compression depth, and a chest compression rate.
